# EUROPÄISCHE PATENTSCHRIFT

(11) **EP 2 585 029 B1**
(45) Veröffentlichungstag und Bekanntmachung des Hinweises auf die Patenterteilung: **12.04.2017**
(21) Anmeldenummer: 11736302.8
(22) Anmeldetag: 24.06.2011
(51) Int. Cl.: A61K 8/19, A61Q 11/00, A61K 33/38, A61K 9/00, A23G 4/00

(54) **ZAHNPFLEGEKAUGUMMI MIT SILBERANTEILEN**
DENTAL CARE CHEWING GUM COMPRISING SILVER MOIETIES
GOMME À MÂCHER DENTAIRE CONTENANT DES CONSTITUANTS ARGENT

(30) Priorität: 25.06.2010 DE 102010030546
(43) Veröffentlichungstag der Anmeldung: 01.05.2013
(73) Patentinhaber: LR Health & Beauty Systems GmbH, 59227 Ahlen (DE)
(72) Erfinder: LARSEN-VEFRING, Sabine, 14089 Berlin (DE)
(74) Vertreter: Maikowski & Ninnemann Patentanwälte Partnerschaft mbB
(86) Internationale Anmeldenummer: PCT/EP2011/060632
(87) Internationale Veröffentlichungsnummer: WO 2011/161247

(56) Entgegenhaltungen:
- EP-A1- 2 042 160
- EP-A1- 2 332 514
- EP-B1- 1 658 040
- WO-A1-93/00884
- WO-A1-98/22427
- DE-T2- 69 106 838
- GB-A- 468 773
- JP-A- 59 152 330
- US-A1- 2007 104 829

## Beschreibung

Die vorliegende Erfindung betrifft einen Silberhaltigen Zahnpflegekaugummi nach Anspruch 1 und Verfahren zu deren Herstellung nach den Ansprüchen 12 oder 13.

Die Herkunft der Kaugummis liegt ursprünglich in Nordamerika. Kaugummis auf der Basis von Komprimaten sind als Zuckerwaren ursprünglich aus dem Bereich Lebensmittel entstanden. Man genießt einen Kaugummi wie jede geschmacksintensive Zuckerware durch Kauen im Mund bis nur noch ein plastischer, geschmacksfreier Anteil zurückbleibt. Diese Kaugummimasse ist nicht zum Verzehr bestimmt. Sie wird nach dem Genuss aus dem Mund entfernt und verworfen. Bei dieser Masse handelt es sich um eine unlösliche Masse. Bei Verzehr von Kaugummis löst sich mit dem Speichel zunächst der Zucker. Nach ca. 15 Minuten ist die Kaugummimasse zuckerfrei, hat ca. 20% Feuchtigkeit aufgenommen und ist dadurch plastisch weich geworden. Sie könnte stundenlang weitergekaut werden und wird anschließend ausgespien.

Ein Kaugummi ist gewöhnlich eine Zuckerware, bestehend aus 50-60%Puderzucker, 15-20% Stärke, 20-25% Kaumasse sowie Aromastoffen. Als sog. wasserunlösliche Kaumasse, auch Gumbase, Kaubase, Gummibase, Kaugummibase genannt, werden Naturstoffe und/oder synthetische Stoffe verwendet. Die Base ist der eigentliche "Gummi", auf dem man kaut. Die natürliche Kaumasse besteht aus Harzen, Kautschuk und Milchsaft bestimmter Bäume, die vorwiegend in Südamerika, Indonesien und Malaysia wachsen. So gewinnt man in Südamerika beispielsweise aus dem Saft des Sapotillbaumes das "Chicle", eine altbewährte Grundlage für Kaugummi. Der eingedickte Saft wird in festen Blöcken zur Weiterverarbeitung zu den Herstellern geschickt. Dort werden natürliche und künstliche Rohstoffe kombiniert, um Kaugummi den bestmöglichen "Biss" zu verleihen.

Kaugummis sind im Allgemeinen aus folgenden Rohstoffgruppen zusammengesetzt: Gum base oder Kaumasse, Weichmacher, Füllstoffe, Gleitmittel, Fette, Emulgatoren, Aromen, Farbstoffe, Antioxidantien und Genuss-Säuren zum Aromatisieren.

Als Kaumasse können entweder Naturstoffe, wie Chicle, Gutta-percha, Latex, Benzoeharze oder Gummi arabicum, oder konsistenzgebende, synthetische Thermoplaste, wie Polyvinylacetat in Mengen von bis zu 65% der Kaumasse, Poly-Butadien-Styrol, Polyisobutylen, Isopren, Polyvinylether und Polyethylen, verwendet werden.

Typische Weichmacher sind Emulgatoren, Harze, Wachse oder Glucitol. Typische Füllstoffe sind Magnesiumstearat, Kreide, Calciumcarbonat, Silicat oder Cellulosen. Die Füllstoffe bzw. technischen Hilfsstoffe erhalten die Rieselfähigkeit bzw. verhindern das Verkleben der Partikel bei niedrigem Druck. Der Anteil an Füllstoffen darf max. 38% betragen. Als Gleitmittel, Fette oder Emulgatoren kommen typischerweise Mineralöle, mikrokristalline Wachse oder pflanzliche Öle zum Einsatz. Diese Hilfsstoffe verhindern das Verkleben der Werkzeuge bzw. an den Werkzeugen.

Kaugummis können aufgrund ihres hohen Zuckeranteils stark kariesfördernd sein, massieren aber auch das Zahnfleisch und die Speicheldrüsen bei Mundtrockenheit. Durch die zugesetzten Aromastoffe wirken Kaugummis auch erfrischend, belebend und/oder durststillend.

Der Trend, zuckerfreie Süßwaren auf den Markt zu bringen, setzte bereits in den 70er Jahren ein. Die Konsumenten werden durch die Auslobungen von Zahnpflegekaugummis wie "nichtkariogen", "medizinisch" und "kosmetisch" angesprochen. In den Anfängen der Herstellung zuckerfreier Kaugummis wurden Zuckeraustauschstoffe der ersten Generation verwendet. Dies sind vor allem Sorbit und Xylit. Sorbit, ab 1950 technisch produziert, ist ein kostengünstiger Zuckeralkohol und ist sehr weit verbreitet. Durch die Entwicklung weiterer Zuckeraustauschstoffe in den 90er Jahren wie Isomalt und Maltit konnten Produkte mit spezielleren Qualitätsmerkmalen hergestellt werden.

Zahnpflegekaugummis dienen der unterstützenden Zahn- und Mundhygiene. Sie sind insbesondere für unterwegs geeignet, wenn keine Möglichkeit zum Zähneputzen besteht. Zahnpflegekaugummis sind gewöhnlich zuckerfrei und enthalten, vergleichbar mit Zahnpasta, Spuren von Mineralien für die Regeneration der Zähne.

Die Gingivia ist ein Teil der Mundschleimhaut und gleichzeitig der periphere Teil des Zahnes. Die Gingivia endet am Zahnhals, umschließt die Zähne und bildet den Epithelansatz. Man unterscheidet die marginale, freie Gingiva mit einer Breite von ca. 1,5mm von der befestigten und der interdentalen Gingiva. Die gesunde Gingiva ist blaßrosa bei hellhäutigen Menschen und bräunlich pigmentiert bei dunkelhäutigen Menschen. Die befestigte Gingiva ist zwischen verschiedenen Individuen und bei verschiedenen Zahngruppen unterschiedlich breit, und wird im Alter breiter. Das für die Gesunderhaltung notwendige Minimum beträgt 2 mm und mehr.

Die häufigsten Erkrankungen des Zahnapparates sind plaquebedingte entzündliche Veränderungen an der Gingiva und am Zahn (Paradont). Eine Gingivitis (Erkrankung der Mundschleimhaut) kann jahrelang bestehen, ohne sich zu einer Paradontitis zu entwickeln. Bei guter Mundhygiene und professioneller Plaque- und Zahnsteinentfernung ist die Gingivitis reversibel.

Maßgeblich für die Entwicklung einer Gingivitis ist einerseits die Bakterienmenge und die Zusammensetzung der Bakterienpopulation bzw. eine individuelle Infektion oder eine bakterielle Invasion. Die Plaquezusammensetzung, d.h. Qualität, Pathogenität, Virulenz, ebenso wie die Plaquemenge sowie die Plaqueretention spielen hier eine Rolle. Eine absolute Plaquefreiheit in der Mundhöhle gibt es nicht. Mit wenig Plaque und einer wenig virulenten Mischflora aus grampositiven, fakultative anaeroben Bakterien und einer intakten Wirtsabwehr kann durchaus eine Gesunderhaltung des Zahnapparates betrieben werden.

Als Plaque werden weiche, weißliche oder gelblich-graue Zahnbelege bezeichnet, die hauptsächlich aus Bakterien bestehen. Die Bakterien sind durch Glykoproteine des Speichels und durch von Bakterien abgesonderte extrazelluläre Polysacchariden, wie z.B. Glucane, Dextrane, Mutane, und Fruktane, wie z.B. Lävane, miteinander unter Ausbildung eines Biofilms verklebt. Plaque lässt sich nicht wegspülen, sondern muss vielmehr mechanisch mit einer Zahnbürste oder anderen geeigneten Instrumenten entfernt werden. Nur aus der Plaque heraus kann sich Karies und der für das Zahnfleisch schädliche Zahnstein entwickeln. Patienten mit starker Plaque tragen ein 5x höheres Risiko, an Paradontitis zu erkranken. Plaque entwickelt sich aus einem den Zahn natürlicherweise als Schutzfilm überziehenden Schmelzoberhäutchen (Pellicle). Auf dieser Schicht setzen sich Mikroroganismen fest und etablieren die sog. Primärflora, aus welcher sich durch Reifung Plaque entwickeln kann. Eine gesunde Gingiva weist eine sehr dünne, adhärente Plaqueschicht von ca. 60µm Stärke auf, die oft nur wenige Zellschichten umfasst.

Auf einem gesäuberten Zahn lagert sich innerhalb von Minuten bis Stunden eine 0,1 bis 0,8µm starkes Schmelzoberhäutchen (Pellicle) aus Speichelglycoproteinen ab. Darauf bilden sich in den ersten 24 Stunden koloniebildende, vorwiegend grampositive Bakterien. Bei Mikroroganismen, die sich als Primärflora auf dem Zahnschmelz etablieren, handelt es sich um ca. 20 verschiedene Bakterienarten, die verantwortlich sind für Zahn- und Zahnfleischerkrankungen differentieller Art.

Insgesamt konnten etwa 300 Spezies und Subspecies aus subgingivalen Plaqueproben isoliert und charakterisiert werden. Nur wenige von ihnen sind parodontpathogen. Zu den grampositiven Mikroorganismen der Plaqueorganismen gehören die folgenden fakultativ anaeroben Keime und anaeroben Keime: Streptococcus wie *S*. *mutans, S*. *sanguis, S*. *salivarius, S. milleri, S. mitis,* Micrococcus, Actinomyces wie *A. naeslundi, A. viscosus,* Bacterionema, Rothia, Nocardia oder Lactobacillus, wie *L. acidophilus, L. casei und L. fermentum.* Der Hauptverursacher für Plaque ist vor allem der Mundhöhlenkeim *Streptococcus mutans,* den es bei der Mundpflege zu reduzieren gilt. Auch der Mundhöhlenkeim *Lactobacillus ssp.* ist an der Bildung von Plaque beteiligt. Diese Plaquebakterien verstoffwechseln die Zucker und bilden Milchsäure. Die Milchsäure löst Mineralien aus dem Zahnschmelz und verursacht Mundgeruch.

Bei der Nahrungsaufnahme werden mit dem Speichel Verdauungsenzyme bereitgestellt, die bereits im Mund mit der Aufspaltung von Nährstoffen beginnen. So werden Kohlenhydrate zu niedermolekularen Zuckern gespalten. Diese Einfachzucker sind Substrat für die im Mund vorhandenen Plaquebakterien (Milchsäurebakterien).

Zahnpflegekaugummis, eingesetzt nach der Nahrungsaufnahme, bewirken, dass der Speichelfluss aufrechterhalten bleibt. Speichel ist leicht basisch und kann die Milchsäure neutralisieren. Dadurch, dass die Kaugummis zuckerfrei sind, werden den Bakterien keine weiteren Nährstoffe für die Milchsäurebakterien zugeführt. So wird auch keine weitere Milchsäure produziert.

Silber, insbesondere hochreines, ionenfrei hergestelltes und hochporöses Silber, weist die Fähigkeit auf, verschiedene Bakterien, Viren und Pilze zu hemmen bzw. zu inaktivieren. Aufgrund seiner antibakteriellen und antiviralen Wirkung findet Silber zunehmend Verwendung in der Behandlung von äußeren Hauterkrankungen oder zur Reduzierung von Schweißgeruch, der üblicherweise durch bestimmte Bakterienarten hervorgerufen wird. So sind derzeit silberhaltige Cremes, Lotionen, Deos aber silberhaltige Textilien erhältlich.

Die Verwendung von Silber in Zahnpflegekaugummis ist ebenfalls bekannt.

So betrifft US 2009/0047340 A1 eine Zusammensetzung umfassend kolloidales Silber in Form von Nanopartikeln sowie Glutathion und Arginin, wobei die Zusammensetzung in Form eines Kaugummis verabreicht werden kann. JP 591 523 30 A betrifft eine Zusammensetzung zur Behandlung von Nikotinsucht, die als wesentlichen Bestandteil metallisches Silber in Form von Pulver, Folien, Streifen oder Stücken sowie eine silberhaltige Legierung mit einem Edelmetall aufweist. Diese Zusammensetzung kann in Form eines Bonbons oder Kaugummis verabreicht werden. Auch in der GB 468, 773 ist die Verwendung von neue Seite
metallischem Silber in einer Kaugummizusammensetzung beschrieben. Aus der US 5,437,858 ist ein Mundreinigungsmittel in Form einer Wasserstoffperoxid-Lösung stabilisiert durch kolloidales Silber bekannt, die unter anderem in Form eines Kaugummis zur Zahnpflege verwendet werden kann. Ein Körperpflegemittel u.a. in Form eines Zahnreinigungskaugummis mit einer Kombination aus einer spezifischen Silberform und Zink wird in EP 1 658 040 B1 beschrieben.

Auch sind Zusammensetzungen zur Verabreichung in Form von Kaugummi, Pulver Flüssigkeiten oder Tabletten beschrieben, die Silber in Form von Nanopartikeln enthalten (US 2006/0251731 A1, US 2009/0047340 A1). Die Verwendung von nanoskaligen Silber- oder Silberverbindungen wurde jedoch in letzter Zeit als für die Gesundheit risikobehaftet eingestuft.

Die im Stand der Technik beschriebenen silberhaltigen Zahnpflegekaugummis weisen jedoch entweder Silber in einer sehr groben Form mit einer geringen Oberflächengröße oder in nanoskaliger Form auf, oder enthalten neben der Silberkomponente weitere Zusatzstoffe wie Zink, die ungewünschte Nebeneffekte hervorrufen können.

Der vorliegenden Erfindung liegt somit die Aufgabe zugrunde, einen Zahnpflegekaugummi zur Verfügung zu stellen, der neben einer hochwirksamen Silberkomponente und den üblichen Inhaltsstoffen auf weitere, insbesondere antibakterielle oder antivirale Komponenten verzichtet.

Diese Aufgabe wird mit einem Zahnpflegekaugummi gemäß Anspruch 1 gelöst.

Demnach umfasst der Zahnpflegekaugummi Silber, welches bevorzugt hochrein, ionenfrei hergestellt, leicht infiltrierbar und hochporös ist. Das zum Einsatz kommende Silber ist durch eine große wirksame Oberfläche gekennzeichnet und liegt typischerweise in Form eines Agglomerat oder Konglomerat von singulären Silberpartikeln vor.

Der erfindungsgemäße Zahnpflegekaugummi umfasst Silber mit einer durchschnittlichen Porengröße zwischen 1 bis 100 µm. Der erfindungsgemäße Zahnpflegekaugummi weist auch kein Zink in metallischer oder ionischer Form auf, und ist demnach frei von Zink. Weitere Inhaltsstoffe sind mindestens ein Abrasivstoff, mindestens ein Zuckeralkohol, Gum base und mindestens ein aromatischer Stoff.

Bevorzugt weist das das eingesetzte Silber eine durchschnittliche Porengröße zwischen 5 bis 50 µm, insbesondere bevorzugt von 10 µm auf. Aufgrund der Porosität ist die wirksame Oberfläche der Silberpartikel besonders groß, so dass die Wirksamkeit der Silberbestandteile des Kaugummis erhöht ist.

Das zum Einsatz kommende Silber kann als ein Agglomerat oder Konglomerat von singulären Silberpartikeln beschrieben werden, wobei die Größe der Silberpartikel bzw. Aggolmerate zwischen 1 bis 200 µm, bevorzugt 5 bis 100 µm, insbesondere bevorzugt 5 bis 20 µm beträgt. Somit liegt die Partikelgröße außerhalb des nanoskaligen Bereiches, so dass mögliche Gesundheitsrisiken vermieden werden.

Des Weiteren enthält der Kaugummi bevorzugterweise keine freien Silber-Ionen. Die verwendeten Silberagglomerate basieren somit ausschließlich auf hochreinem, metallischem Silber. Es ist jedoch möglich, dass aus dem metallischen Silber Silber-Ionen durch Wechselwirkung mit den Flüssigkeiten des Mundraumes freigesetzt werden können.

Der Gehalt an Silber im Zahnpflegekaugummi kann zwischen 0,01 bis 1 Gew%, bevorzugt 0,01 bis 0,5 Gew%, insbesondere bevorzugt zwischen 0,01 und 0,1 Gew% betragen. Ganz besonders bevorzugt ist ein Gehalt an 0,05 Gew% Silber.

In einer weiteren Ausführungsform umfasst der Kaugummi Pantothensäure (Vitamin B5) oder mindestens ein Derivat davon. Insbesondere ist der Einsatz von Dexpanthenol bevorzugt. Dexpanthenol ermöglicht eine gute Verteilung der Silberagglomerate bzw. Silberpartikel in der Kaumasse und wirkt darüber hinaus wie auch Vitamin B5 entzündungshemmend, juckreizlindernd und wundheilungsfördernd.

Der Zahnpflegekaugummi kann ebenfalls mindestens ein Schleifmittel oder mindestens einen Abrasivstoff, insbesondere Calciumcarbonate, Calciumphospate, Metaphosphate, Kieselsäure, Aluminiumoxide, Silikate und/oder Talkum, umfassen.

Des Weiteren sind mindestens ein Suspensionsstoff oder Feuchthaltemittel, insbesondere Wasser, Glycerin, Propylenglykol und/oder Sorbitsirup, sowie mindestens ein Verdickungs-, Stabilisierungs- und/oder Bindemittel, insbesondere Gele, Stärken, Alginate, Öle und/oder Cellulose Gum, im Kaugummi enthalten.

Zur Verbesserung der Geschmackseigenschaften können dem Kaugummi mindestens ein aromatischer Stoff, mindestens ein Süßungsmittel und/oder mindesten ein Zuckeraustauschstoff, insbesondere Menthol, Pfefferminzöl, Natriumsaccharin, Aspartam, Acesulfam, Sorbit, Malit, Xylit und/oder Fructose, zugegeben werden.

Weitere chemische Additive, Farbstoffe und/oder pH-Regulatoren, insbesondere Fluoride, Adstringentien, Entzündungshemmer, Desensibilisierungsmittel, Vitamine, Panthenol, Weißpigmente und/oder Natriumhydroxid, können im Kaugummi ebenfalls enthalten sein.

Die quantitative Zusammensetzung des Zahnpflegekaugummis kann je nach Geschmack und Anforderzungen variieren. So kann der Zahnpflegekaugummi folgende allgemeine Zusammensetzung umfassen: 20 - 30 Gew% Gum Base, 40 - 60 Gew% Sorbitol-Pulver, 15 - 25 Gew% 70%ige Sorbitlösung, 15 - 25 Gew% Xylitol, 1 - 5 Gew% Calciumcarbonat, 0,01 - 1 Gew % Aspartam, 0,1 - 3 Gew% Magnesiumstearat, 0,01 - 1 Gew% Acesulfam, 1 - 5 Gew% Aromen, 1 - 10 Gew% Calciumphosphat, 0,1 - 5 Gew% Talkum, 0,01 - 1 Gew% Mikrosilber, 1 - 5 Gew % Siliziumdioxid, 0,1 - 1 Gew% Pflanzenöl, 1 - 5 Gew% Glycerin und 0,1 - 5 Gew% Dexpanthenol.

Das verwendete Silber basiert auf der in der EP 1 658 040 B1 beschriebenen Silberzusammensetzung. Im Unterschied zu dieser bekannten Silberzusammensetzung wird jedoch eine zinkfreie Qualität des Silberrohstoffes eingesetzt. Im Rahmen einer möglichst geringen toxikologischen Belastung ist es erstrebenswert, auf eine zusätzliche Belastung der Mundhöhle durch Zink zu verzichten. So sind in der Vergangenheit durch im Lebensmittelbereich eingesetztes Zink hervorgerufene Krankheitsbilder beschrieben worden. Kartoffelsalat in Zinkwannen, Limonade in galvanisch verzinkten Eisenkannen, Apfelmus in Zinkgefäßen oder auf verzinkten Drahtsieben getrocknete Früchte führten zu Erbrechen, Übelkeit, Durchfällen und Leibschmerzen. Durch Einwirken von organischen Säuren bildeten sich in den beschriebenen Fällen leichtlösliche Salze wie Zinkchlorid sowie Zinksulfat mit reizender Wirkung auf den Magen-Darm-Trakt sowie ggf. eiweißfällender Wirkung in höheren Konzentrationen. Auch Austern enthalten in manchen Meeresgebieten ungewöhnlich hohe Mengen Zink. Zur Vermeidung einer zusätzlichen gesundheitlichen Belastung wird daher auf den Einsatz von Zink in den erfindungsgemäßen Zahnpflegekaugummis verzichtet.

Silber wird als chemisches Element mit *Argentum* mit dem chemischen Symbol Ag (CAS Nr. 7440-22-4, EINECS No: 231-131-3) bezeichnet. Es ist ein Edelmetall aus der 1. Nebengruppe des Periodensystems mit der Ordnungszahl 47 und einem Atomgewicht von 107,8682. Bei der als Silber-Komponente verwendeten Silberzusammensetzung handelt es sich um ein hochreines, ionenfrei hergestelltes und leicht infiltrierbares hochporöses Silber von der durchschnittlichen Porengröße 10 µm mit den folgenden Produkteigenschaften:

**Tabelle 1: Eigenschaften des verwendeten Silbers**

| **Pulvereigenschaften** | **Methode** | **Wert** | **Einheit** |
|---|---|---|---|
| Spezifische Oberfläche | N₂-Adsorption nach BET (volumetrisch) | 3-5 | M²/g |
| Mittlere Agglomeratsgröße (d50) | Laser Granulometrie | 5-20 | µm |
| Verhältniszahl (d10/d90) | Laser Granulometrie | 4-15 | |
| Klopfdichte | | 0,5-0,7 | g/cm³ |
| Spezifische dc-Leitfähigkeit | Gemessen bei Klopfdichte 20 °C | <500 | [Ωcm]⁻¹ |
| Restfeuchtegehalt | | 0,1-0,2 | Gew.-% |
| Chemische Elemente | | | |
| Silbermetallbasis | AAS | 99,9 | % |
| Wolfram (gebunden als WO₃) | AAS | <500 | ppm |
| Cu | AAS | <250 | ppm |
| O total | Gemessen mit Heißextraktion (HE) | <0,6 | % |
| C | HE | <0,1 | % |
| Na, K, Cl | AAS | Y5 | ppm |

Aufgrund der spezifischen Zusammensetzung des erfindungsgemäßen Zahnpflegekaugummis wird eine Reduktion der Keimzahlen der Hauptkeime *Streptococcus mutans* und des Mundhöhlenkeimes *Lactobacillus ssp.* bewirkt. Dieser keimreduzierende Effekt beruht insbesondere auf dem Zusammenwirken von Zuckeraustauschstoffen, Microsilber und Abrasivstoffen.

Der Zahnpflegekaugummi besitzt somit eine Wirkung, die einen großen Einfluß auf die Zahn-und Zahnfleischhygiene hat. Die Reduktion dieser Keime auf die Normwerte ist optimal, um die Entstehung von Plaque zu verhindern. Das Produkt ist anwenderfreundlich und effektiv besonders unterwegs und in Momenten, in denen kein Wasser und keine Zahnbürste zur Verfügung stehen.

Bei zuckerfreien Kaugummis wie im Fall des vorliegenden Zahnpflegekaugummis werden anstelle von Zucker bevorzugt Süßungsmittel, insbesondere Acesulfam oder Aspartam, eingesetzt.

Acesulfam ist ein Derivat der Acetessigsäure. Es ist stabil, sehr gut wasserlöslich und besitzt etwa die 200fache Süßkraft von Zucker. Es wird nicht metabolisiert, d.h. Acesulfam wird resorbiert und unverändert mit dem Urin ausgeschieden. Es ist nicht kariogen. Mit Aspartam zeigt sich eine synergistische Wirkung. Die Süßungskraft wird verstärkt bis zu 40%. Acesulfam wird schnell resorbiert und vollständig mit dem Urin wieder ausgeschieden.

Aspartam ist ein Dipeptid der beiden Aminosäuren L-Asparaginsäure und L-Phenylalanin. Nur die α-Form des L-L-Peptides weist eine Süßungskraft auf. Während der Verdauung wird Apartam im Gastrointestinaltrakt in Asparaginsäure, Phenylalanin und Methanol zerlegt. Die Resorption aller Abbauprodukte verläuft rasch und vollständig. Die Aminosäuren werden wie Nahrungsproteine verstoffwechselt, Methanol zu CO₂ oxidiert. Spuren von Diketonpiperazin werden im Urin ausgeschieden. Die primäre Aufgabe von Aspartam besteht in der Komplementärsüße der durch Zuckeraustauschstoffe verminderten Süßkraft. In Kaugummis kann der Geschmack und die Dauer der Süßwirkung im Vergleich zu zuckergesüßten Produkten verlängert werden.

Bei Zuckeraustauschstoffen handelt es sich in der Regel um Zuckeralkohole und damit um mehrwertige Alkohole. Sie werden durch katalytische Hydrierung der entsprechenden Zuckerarten hergestellt. In der Natur kommen sie in kleinen Mengen auch in Früchten, in einigen Gemüsesorten und in einigen Holzarten vor. Diese Austauschstoffe werden in der Kaugummiherstellung eingesetzt, da sie eine hohe chemische Stabilität besitzen, thermisch stabil sind, eine feuchtigkeitsstabilisierende Wirkung besitzen, in hohen Konzentrationen eine konservierende Wirkung aufzeigen und eine hohe mikrobielle Stabilität aufweisen.

Zuckeralkohole werden generell nur teilweise verstoffwechselt und aufgenommen. Gelangen größere Mengen in den Dickdarm, werden die Zuckeralkohole hier durch die vorhandene Mikroflora fermentiert. Zuckeralkohole haben aufgrund ihrer langsameren Resorption einen niedrigen Brennwert, der von der EU auf 2,4kcal/g festgelegt wurde. Kaugummirezepturen erhält durch mehrwertige Zuckeralkohole eine positive Beeinflussung des Härtegrades der Kaumasse, verbesserte sensorische Eigenschaften wie lang anhaltenden Geschmack durch intensivere Aromenbindung. Sie finden Einsatz in zuckerfreien Zuckerwaren, sind zahnfreundlich, besitzen karieshemmende Wirkung und verminderte Plaquebildungen.

Sorbit/Sorbitol zählt zur Gruppe der sechswertigen Zuckeralkohole und wird durch katalytische Reduktion von Glucose hergestellt. Sorbit weist durch seine negative Lösungswärme sensorisch einen Kühleffekt auf. Sorbit wird gegenüber Zucker wesentlich langsamer zu organischen Säuren abgebaut. Sorbit wird insulinunabhängig abgebaut, woraus dessen Eignung für Diabetiker resultiert. Sorbit ist ein Wasserstabilisator durch begrenzte Hygroskopie und kann Komplexbildner für Spurenelemente sein. Sorbit ist nicht wasserdampfflüchtig.

Xylitol als fünfwertiger Zuckeralkohol gewinnt man durch katalytische Hydrierung der Xylose. Xylit weist ebenso wir Sorbit sensorisch einen Kühleffekt auf. Xylit wird durch Mikroorganismen in der Mundhöhle in der Regel nicht vergoren. Xylit wird im menschlichen Organismen insulinunanhängig verwertet und ist daher Diabetikergeeignet. Malitol ist ein Disaccharidalkohol und wird gewonnen durch Reduktion aus Maltose. Xylit besitzt dieselbe Süßungskraft wie Zucker, Sorbit und Maltit dagegen sind geringfügig weniger süß. Ebenso wie Sorbit und Xylit wird Malitol im menschlichen Organismen insulinunanhängig verwertet und ist daher geeignet für Diabetiker.

Seit den 70er Jahren des letzten Jahrhunderts ist der Anteil an zuckerfreien Kaugummis stark gestiegen. Die Herstellung von zuckerfreien Kaugummis weicht prinzipiell von der von zuckerhaltigen Kaugummis ab. Die Hauptkomponente Zucker wurde anfangs durch den Zuckeralkohol Sorbit, später durch Xylit und Mannit, seit ca. 1987 auch durch Isomalt ersetzt. Der flüssige Anteil besteht meistens aus 70 %igem nicht kristallisierenden Sorbitsirup. Sowohl der Anteil der Gum Base, als auch der der Aromen und Weichmacher (Glycerin) ist höher als im zuckerhaltigen Kaugummi. Die Kaubase hat einen Anteil von ca. 20 % bis 30 % bzw. 25 bis 30 Gew% im Gegensatz zu ursprünglich 18-20 % im herkömmlichen Kern. Zusätzlich müssen andere Kaubasenqualitäten verwendet werden. Zuckerfreie Kaugummis sind lagerungsempfindlicher als Zuckerkaugummis. Sie neigen stärker zur Austrocknung. Dies ist auf den flüssigen Bestandteil, den Sorbitsirup, zurückzuführen. Die Ursache liegt in der geringeren Gleichgewichtsfeuchte des Sorbitsirups im Vergleich zum Glucosesirup.

Im Fall der vorliegenden Zahnpflegekaugummis ermöglicht die Verwendung von pulverförmigem Sorbitol jedoch die Herstellung einer feuchtestabileren Zusammensetzung. Auch die gezielte Verwendung von Glycerin führt zur Verbesserung der Lagerstabilität.

Generell ergeben sich Qualitätsmängel durch zu schnelles Austrocknen bei ungeeigneter oder undichter Verpackung sowie oxidativer Veränderung mit Aromaverlust durch zu langes Lagern. Bei optimalen Lagerbedingungen, d.h. 10-20 °C, 70-80% relative Luftfeuchte, beträgt die Haltbarkeit für undragierte Kaugummis 4 Monate, für dragierte Kaugummis 6 Monate und für Silberhaltige Kaugummis mindestens 18 Monate. Die längere Haltbarkeit der erfindungsgemäßen silberhaltigen Kaugummis ergibt sich insbesondere aus der Feuchthaltewirkung von Sorbitol und Glycerin einerseits sowie der konservierenden Wirkung des Silbers andererseits, da je höher die Wasserbindungskapazität eines Lebensmittels ist, um so höher ist auch seine Anfälligkeit gegen Mikroorganismen.

Kaugummis können in verschiedenen Darreichungsformen angeboten werden. So sind Kaugummis in Form von Würfeln, Kugeln, Riegeln, flach geschnittenen, undragierten Scheiben, in Staubzucker gewalzt, und einzeln verpackt, als Granulat oder Spray auf dem Markt. Die Würfel werden in so genannten Toffeeherstellungsanlagen produziert. Die geformten und verpackten Stücke in Kissenform werden mit einer aufdragierten Zuckerdecke versehen. Kugeln sind ebenfalls mit einer Zuckerdecke überzogen zum Schutz vor Austrocknung. Riegel werden auch Streifen, stick gum oder Scheiben genannt.

Je nach Anteil des Weichmachers und der Gummistoffe eignen sich die Kaugummis zum Aufblasen (bubble gum) oder nur zum Kauen. Kaugummibasen enthalten im Gegensatz zu Blasgummibasen viel Wachs (Microwachs) und wenig Latex.

Der vorliegende Zahnpflegekaugummi kann nach einem Verfahren mit den folgenden Schritten hergestellt werden:
- Vermischen der pulverförmigen und flüssigen Inhaltsstoffe, insbesondere in einem Konusmischer,
- Kneten des Gemisches bis zur Homogenität, bevorzugt bis zu einer Endtemperatur von 45 °C,
- Extrusion des Gemisches, und
- Konfektionierung.

Demnach werden zur Herstellung die Rohstoffe zunächst abgewogen und gemischt. Das Vermischen der Zutaten erfolgt in der Regel in einem Konusmischer. Die Pulvermischung wird von unten in der Mitte nach oben befördert, von wo aus es wieder nach unten fällt. Hierzu kreisen schräggestellte Förderschnecken entlang der schrägen Wandung des Konusmischers. Flüssige Komponenten werden aufgesprüht.

Zur Herstellung eines Kaugummis wird in der Regel eine Kaugummibase in einem Wärmeschrank bei z.B. 55°C erwärmt, bevor sie auf einen Kneter gegeben wird. Anschließend wird die Kaumasse unter Zugabe weiterer pulverförmiger Zutaten in der vorgeschriebenen Reihenfolge weiter geknetet. Danach werden Aroma, Sorbitsirup und Glycerin zugegeben. Geknetet wird, bis die Masse bei einer Endtemperatur ca. 45°C homogen ist. Die Masse wird portioniert und 15-20 Minuten anteilig auf einer mit Talkum bestreuten Unterlage zwischengelagert (Reifezeit), mit einem geeigneten Extruder extrudiert und wie üblich weiterverarbeitet. Danach erfolgt die Kühlung und Konfektionierung wie z.B. Schneiden, Wickeln und Verpacken.

Der Zahnpflegekaugummi der vorliegenden Erfindung kann auch in Form von Komprimaten hergestellt werden. Komprimate werden aus Pulver zu kleinen, formstabilen Stücken gepresst. Komprimate sind in vielfältigen Formen herstellbar. Sie sind in der Regel kleinstückig, hart und flach. Komprimate werden teilweise auch als Tabletten oder Pastillen bezeichnet. Eine klare Abgrenzung zwischen diesen ist nicht möglich. Die Konsistenz reicht von weich, leicht zerfallend bis zu extrem hart und glatt.

Das Verfahren zur Herstellung eines erfindungsgemäßen Zahnpflegekaugummis umfasst die folgenden Schritte:
- Vermischen der pulverförmigen und flüssigen Inhaltsstoffe, insbesondere in einem Konusmischer,
- Einfüllen des Pulvers in eine Matrize,
- Verpressen des Pulvers in der Matrize,
- Lösen der komprimierten Masse aus der Matrize unter Verwendung von mindestens einem Trennmittel, und
- Konfektionierung.

Demnach werden die in einem ersten Schritt die Rohstoffe bzw. Inhaltsstoffe der Zusammensetzung abgewogen und gemischt Das Vermischen der Zutaten erfolgt in der Regel in einem Konusmischer. Die Pulvermischung wird von unten in der Mitte nach oben befördert, von wo aus es wieder nach unten fällt. Hierzu kreisen schräggestellte Förderschnecken entlang der schrägen Wandung des Konusmischers. Flüssige Komponenten, wie Wasser, Sorbitlösung oder Glycerin werden aufgesprüht.

Komprimate werden aus Pulvermischungen hergestellt, die in eine Matrize gefüllt wird. Direkttablettierbare Pulver werden sofort gemischt und anschließend verpresst, während Pulver, die sich nicht direkt verpressen lassen, zuvor vorbereitet werden müssen, z.B. durch Feuchtgranulation oder thermische Behandlung. Die Herstellung der Tabletten erfolgt üblicherweise in Excenterpressen. Excenterpressen bauen einen hohen Druck auf.

Die vorliegenden Zahnpflegekaugummis werden jedoch bevorzugt in einer Rundläufertablettenpresse verpresst. Rundläufer haben üblicherweise Wechselrotoren. Dadurch kann bei Verschmutzungen schnell ein Rotortausch veranlasst werden und damit die Produktion fortgesetzt werden. Andererseits kann auch ein Formatwechsel in kürzester Zeit vorgenommen werden. Auf der Kreisbahn eines Rotors erfolgen die Einzelschritte des Matrizenbefüllens, des Verdichtens und des Auswerfens in kontinuierlicher Weise hintereinander. In der Matrize befindet sich ein Presswerkzeug (Unterstempel). Der Unterstempel wird nach unten geführt. Das obere Presswerkzeug setzt auf und Druckrollen drücken das obere und untere Presswerkzeug aufeinander. Das Pulver wird so zu einem Komprimat zusammengepresst. Haben sich Ober- und Unterstempel gelöst, wird das Komprimat mit einem Abstreifer aus der Matrize geschoben.

Zur Lösung der komprimierten Masse aus der Matrize werden als Trennmittel bevorzugt Fette und Öle, insbesondere Kokosöl, verwendet. Die Matrizen werden mit einem geeigneten Trennmittel besprüht oder bestäubt.

Die vorliegende Erfindung wird anhand der folgenden Ausführungsbeispiele in Kombination mit den Figuren zur besseren Verständlichkeit näher beschrieben. Es zeigen:
- Figur 1: ein erstes Diagramm mit den Ergebnissen von mikrobiologischen Untersuchungen, und
- Figur 2: ein zweites Diagramm mit den Ergebnissen von mikrobiologischen Untersuchungen.

Bevorzugte Kaugummizusammensetzung sind der folgenden Tabelle anhand der Beispiele 1 bis 3 zu entnehmen.

**Tabelle 2: Bevorzugte Zusammensetzungen des Zahnpflegekaugummis**

| **Zutat** | **Beispiel 1: Anteil in [%]** | **Beispiel 2: Anteil in [%]** | **Beispiel 3: Anteil in [%]** | **Beispiel 4: Anteil in [%]** |
|---|---|---|---|---|
| Gum Base | 23,85 | 21,85 | 25,0 | 21,85 |
| Sorbitol (Pulver) | 42,56 | 41,54 | 58,5 | 58,5 |
| Sorbitlösung (70%) | - | - | 12,0 | 12,0 |
| Xylitol | 19,47 | 19,31 | - | - |
| Calciumcarbonat | 2,80 | - | - | - |
| Aspartam | 0,20 | 0,20 | - | - |
| Magnesiumstearat | 1,00 | 1,50 | - | - |
| Acesulfam | 0,07 | 0,07 | - | - |
| Aromen | 2,20 | 2,20 | 1,5 | 1,5 |
| Calciumphosphat | 6,80 | 6,80 | - | - |
| Talkum | 1,00 | 1,00 | - | - |
| Microsilber | 0,05 | 0,05 | 0,05 | 0,05 |
| Siliciumdioxid | - | 2,43 | - | - |
| Pflanzenöl | - | 0,25 | - | - |
| Glycerin | - | - | 2,95 | 2,95 |
| Dexpanthenol | - | - | - | 3,15 |

Zum Nachweis der Wirksamkeit des Zahnpflegekaugummis wurde ein klinischdermatologischer Anwendungstest durchgeführt.

Ziel der Untersuchung war es, die Wirkung des Produktes Zahnpflegekaugummi mit Microsilber und eines handelsüblichen Zahnpflegekaugummis (Placebo) zu untersuchen.

Als Placebo wurde ein Kaugummi mit dem Namen Wrigley's Extra (Peppermint) ohne Zucker mit einer Länge von 4,5cm, einer Breite von 1,2 cm und einem Gewicht von ca. 2g verwendet. Der Placebo weist die Inhaltsstoffe Sorbitol, Gum Base, Xylitol, Glycerin, Aroma, Mannitol, Hydrogenated Starch Hydrolysate, Lecithin, Aspartame, BHA auf und enthält eine Phenylalaninquelle.

Bei den männlichen und weiblichen Probanden wurde vor und nach der einmaligen Anwendung der Produkte zu verschiedenen Zeitpunkten der Bakterienstatus in der Mundhöhle durch eine Speichelprobe ermittelt. Vor Beginn des Anwendungstestes wurden alle Testpersonen dermatologisch untersucht. Es befanden sich nur Teilnehmer/-innen in der Prüfgruppe, bei denen keine pathologische Haut- und Schleimhautveränderungen vorlagen. Jede Testperson benutzte das jeweilige Produkt gemäß Anwendungshinweis. Bei Bedarf konnten die Probanden jederzeit den testbegleitenden Dermatologen bei objektiven oder subjektiven Haut- oder Schleimhautveränderungen zu Rate ziehen. Insgesamt waren 20 Probanden beteiligt, davon 12 weibliche und 8 männliche. Das Alter der Probanden lag zwischen 26 und 54 Jahren.

Der Anwendertest erfolgte an zwei aufeinanderfolgenden Tagen. An Tag 1 erfolgte die Verabreichung des Zahnpflegekaugummis mit Microsilber und an Tag 2 des PlaceboKaugummis. Der Tagesablauf umfasste ein normales morgentliches Zähneputzen. Das gemischte Mittagessen wurde zwischen 11 und 12 Uhr eingenommen. Um 12.30Uhr erfolgte die erste Probennahme Tag 1 (Probe 1-1) bzw. Tag 2 (Probe 2-1). Um 13 Uhr wurde ein zuckerhaltiges Bonbon gelutscht. Nach Auflutschen des Bonbons erfolgte die zweite Probenahme Tag 1 (Probe 1-2) bzw. Tag 2 (Probe 2-2). Um 13.20Uhr wurde das tagesspezifische Kaugummi für 10 Minuten gekaut. Direkt nach dem Ausspucken des Kaugummis erfolgte die dritte Probennahme Tag 1 (Probe 1-3) bzw. Tag 2 (Probe 2-3). Um 14.30 Uhr schlossen sich eine vierte Probenahme Tag 1 (Probe 1-4) bzw. Tag 2 (Probe 2-4), um 15.30Uhr eine fünfte Probenahme Tag 1 (Probe 1-5) bzw. Tag 2 (Probe 2-5) und um 17.30Uhr ein sechste Probenahme Tag 1 (Probe 1-6) bzw. Tag 2 (Probe 2-6) an. In dem Zeitraum zwischen 13 Uhr und 17.30Uhr durfte nur kohlensäurefreies Wasser zu sich genommen werden.

Vor Beginn der Untersuchungen zeigten alle 20 Testpersonen eine gesunde Haut, ein gesundes Zahnfleisch und eine erscheinungsfreie Mundschleimhaut. Pathologische Hautveränderungen oder Mundschleimhautveränderungen waren in keiner Form festzustellen.

Auch im Verlauf des Testes zeigten die 20 Testpersonen keinerlei pathologische Haut-, Zahnfleisch- oder Mundschleimhautveränderungen. Hautfachärztliche Behandlungen waren in keinem Fall notwendig.

Bei der dermatologischen Abschlußuntersuchung nach dem Testende zeigten sich bei keiner der 20 Testpersonen pathologische Hautveränderungen oder Mundschleimhautveränderungen. Die genannten Präparate wurden von 20 Testpersonen gut vertragen und führten bei diesem Kollektiv zu keinen pathologischen Hautveränderungen.

Die Testpersonen beurteilten die beiden Produkte, silberhaltiger Zahnpflegekaugummi und Placebo, anhand eines Fragebogens nach Geschmack, Wirkung und Verträglichkeit des Produktes. Zusammenfassend kann festgestellt werden, dass aus dermatologischer Sicht der Zahnpflegekaugummi mit Mikrosilber bei einmaliger Anwendung nach klinischdermatologischen und subjektiven Kriterien von 20 Testpersonen sehr gut vertragen wurde.

Zur Durchführung der mikrobiologischen Untersuchungen wurden 5 ml Speichel gesammelt, homogenisiert und als Kulturvorlage genutzt. Als Nährplatten dienten standardisierte "Streptococcus Platten". Für den Lactobacillus wurden "Rogosa Platten" genutzt. Die gemessene Einheit ist KBE/ml.

Die Ergebnisse der mikrobiologischen Untersuchungen sind in den folgenden Tabellen zusammengefasst.

**Tabelle 3: mikrobiologische Ergebnisse für Streptococcus mutans**

| | **Silberhaltiger Kaugummi Tag 1 [KBE/ml]** | **Silberhaltiger Kaugummi Tag 1 [% von Probe 1-1]** | | **Placebo Tag 2 [KBE/ml]** | **Placebo Tag 2 [% von Probe 2**-**1**] |
|---|---|---|---|---|---|
| Probe 1-1 | 17650 | 100 | Probe 2-1 | 11150 | 100 |
| Probe 1-2 | 9150 | 52 | Probe 2-2 | 6900 | 62 |
| Probe 1-3 | 15600 | 88 | Probe 2-3 | 11350 | 102 |
| Probe 1-4 | 6150 | 35 | Probe 2-4 | 4150 | 37 |
| Probe 1-5 | 6400 | 36 | Probe 2-5 | 4400 | 40 |
| Probe 1-6 | 8400 | 48 | Probe 2-6 | 6650 | 60 |

**Tabelle 4: Mikrobiologische Ergebnisse für Lactobacillus ssp.**

| | **Silberhaltiger Kaugummi Tag 1 [KBE/ml]** | **Silberhaltiger Kaugummi Tag 1 [% von Probe 1-1]** | | **Placebo Tag 2 [KBE/ml]** | **Placebo Tag 2 [% von Probe 2-1]** |
|---|---|---|---|---|---|
| Probe 1-1 | 5050 | 100 | Probe 2-1 | 5050 | 100 |
| Probe 1-2 | 3300 | 65 | Probe 2-2 | 3550 | 70 |
| Probe 1-3 | 4950 | 98 | Probe 2-3 | 5250 | 104 |
| Probe 1-4 | 1800 | 36 | Probe 2-4 | 2000 | 40 |
| Probe 1-5 | 2200 | 44 | Probe 2-5 | 2400 | 48 |
| Probe 1-6 | 2600 | 52 | Probe 2-6 | 2600 | 52 |

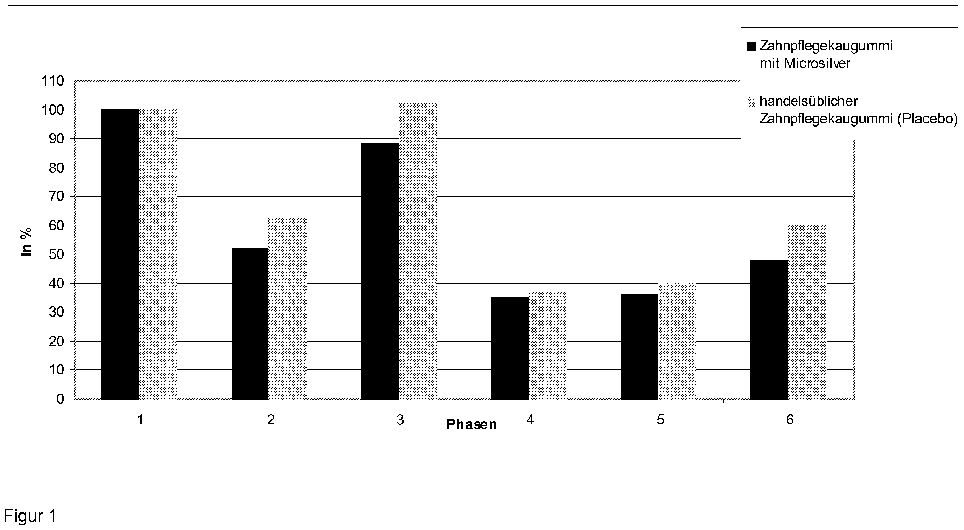

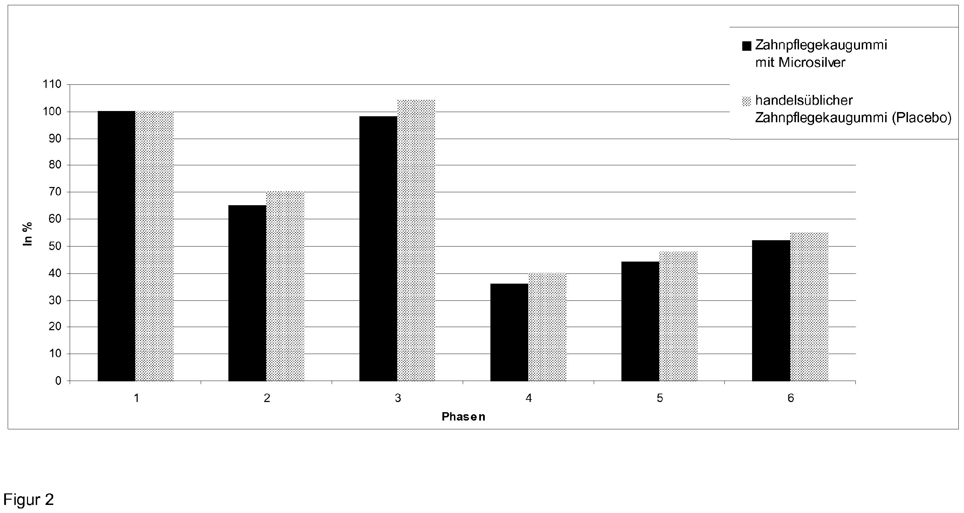

In den Diagrammen der Figuren 1 und 2 sind jeweils die Ergebnisse der mikrobiologischen Ergebnisse für *Streptococcus mutans* (Figur 1) und *Lactobacillus ssp.* (Figur 2) zusammengefasst. Lactobacillen (Stäbchen) und Streptococcus (Koken) sind jeweils grampositive aerobe und fakultativ anaerobe Keime und stellen die Hauptbesiedelung in Besiedelungsphase 1 am Bespiel des supragingivalen Plaque dar.

Die Auswertung der mikrobiologischen Ergebnisse wurde anhand der relativen Keimzahlenentwicklung vorgenommen, in dem die durchschnittliche Keimzahl der Mundflora in der Ausgangssituation nach einem normalen, gemischten Mittagessen auf 100% gesetzt wurde (Probe 1). Die erzielten Keimzahlen (Probe 2) spiegeln den Einfluß eines Bonbons sowie eines danach 10minütig gekauten Kaugummis (Probe 3) und dessen Langzeitwirkung (Probe 4, 5 und 6) nur mit einer Zeitverzögerung dar. Diese Zeitverzögerung entsteht dadurch, dass sofort nach Verzehr des Bonbons durch erhöhten Speichelfluss die Keimzahl zunächst reduziert wird und die vorhandenen Keime erst eine gewisse Zeit zur Vermehrungsinitiation benötigen. Die Keimzahlen in Probe 2 unmittelbar nach Bonbongenuss waren in der Regel geringer als in der Ausgangssituation. In Probe 3 zeigt sich eine gleich hohe bzw. erhöhte Keimzahl gegenüber der Ausgangssituation. Die Keime in der Mundhöhle haben den Zucker verstoffwechselt und zeigen eine signifikante Vermehrungsrate. Der Genuss der Kaugummis macht sich alleine durch den Kauvorgang mikrobiologisch zu diesem Zeitpunkt noch nicht bemerkbar. Die Kaugummis zeigen sofort nach Verzehr und Verwerfen des Kaugummis bzgl. der Keimbelastung der Mundflora noch keine Wirkung. Erst nach einer Reaktionszeit von ca. 30 Minuten sind signifikante Keimreduzierungen in der Mundhöhle auf ca. 30% feststellbar. Diese Keimreduktion fiel beim Silberhaltigen Kaugummi jeweils stärker aus als beim Placebo-Kaugummi. In Zeitabständen von 1 bzw. 2 Stunden steigt die Keimbelastung der Mundhöhle langsam wieder an. Die dauerhafte Wirkung eines einmalig verzehrten Kaugummis ist beim silberhaltigen Kaugummi gegenüber dem Placebo jedoch höher. Der keimreduzierende Effekt ist in den Tabellen 5 und 6 zusammengefasst.

**Tabelle 5: Keimreduzierender Effekt des silberhaltigen Kaugummis auf Lactobacillus ssp.**

| **Probe** | **Silberhaltiger Kaugummi [%]** | **Placebo-Kaugummi [%]** | **Differenz Δ** |
|---|---|---|---|
| 3 | 98 | 104 | 6 |
| 4 | 36 | 40 | 4 |
| 5 | 44 | 48 | 4 |
| 6 | 52 | 55 | 3 |

**Tabelle 6: Keimreduzierender Effekt des silberhaltigen Kaugummis auf Streptococcus mutans**

| **Probe** | **Silberhaltiger Kaugummi [%]** | **Placebo-Kaugummi [%]** | **Differenz Δ** |
|---|---|---|---|
| 3 | 88 | 102 | 14 |
| 4 | 35 | 37 | 2 |
| 5 | 36 | 40 | 4 |
| 6 | 48 | 60 | 12 |

Zusammenfassend kann gesagt werden, dass die erzielte Keimreduktion in der Mundhöhle durch Verzehr eines silberhaltigen Kaugummis höher und lang anhaltender im direkten Vergleich zu einem Placebo-Kaugummi ist. Insofern ist die zahnpflegende Wirkung durch den Anteil an Silber verstärkt.

Die synergistische Wirkung der Zutaten in dem silberhaltigen Zahnpflegekaugummi erzielen eine nachweisliche Keimreduzierung in der Mundhöhle, einen über mind. 5h anhaltenden Effekt und die Wirksamkeit in der Mundhöhle ist gegenüber Placebokaugummis (handelsüblichen Kaugummis ohne Silberanteilen) erhöht und verlängert. Diese synergistische Wirkung bezieht sich auf die gleichzeitige Anwesenheit von abrasiven Schleifkörper, Zuckeralkoholen, Microsilber gemäß der beanspruchten Erfindung.

## Patentansprüche

1. Zahnpflegekaugummi umfassend Silber, mindestens einen Abrasivstoff, mindestens einen Zuckeralkohol, Gum Base und mindestens einen aromatischen Stoff, wobei kein Zink in metallischer oder ionischer Form enthalten ist und das Silber eine durchschnittliche Porengröße zwischen 1 bis 100 µm aufweist.

2. Zahnpflegekaugummi umfassend Silber, mindestens einen Abrasivstoff, mindestens einen Zuckeralkohol, Gum Base und mindestens einen aromatischen Stoff, wobei kein Zink in metallischer oder ionischer Form enthalten ist, **dadurch gekennzeichnet, dass** das Silber in Form eines Agglomerates mit einer Agglomeratgröße zwischen 1 bis 200 µm, bevorzugt 5 bis 100 µm, insbesondere bevorzugt 5 bis 20 µm vorliegt.

3. Zahnpflegekaugummi nach Anspruch 1, **dadurch gekennzeichnet, dass** das Silber eine durchschnittliche Porengröße zwischen 5 bis 50 µm, bevorzugt von 10 µm aufweist.

4. Zahnpflegekaugummi nach einem der vorhergehenden Ansprüche, **dadurch gekennzeichnet, dass** das Silber keine freien Silber-Ionen aufweist.

5. Zahnpflegekaugummi nach einem der vorhergehenden Ansprüche, **dadurch gekennzeichnet, dass** der Gehalt an Silber zwischen 0,01 bis 1 Gew%, bevorzugt 0,01 bis 0,5 Gew%, insbesondere bevorzugt zwischen 0,01 und 0,1 Gew% beträgt.

6. Zahnpflegekaugummi nach einem der vorhergehenden Ansprüche, **dadurch gekennzeichnet, dass** der Kaugummi Pantothensäure (Vitamin B5) oder mindestens ein Derivat davon, insbesondere Dexpanthenol, umfasst.

7. Zahnpflegekaugummi nach einem der vorhergehenden Ansprüche, **dadurch gekennzeichnet, dass** das mindestens eine Schleifmittel oder Abrasivstoff Calciumcarbonate, Calciumphospate, Metaphosphate, Kieselsäure, Aluminiumoxide, Silikate und/oder Talkum umfasst.

8. Zahnpflegekaugummi nach einem der vorhergehenden Ansprüche, **gekennzeichnet durch** mindestens ein Suspensionsstoff oder Feuchthaltemittel, insbesondere Wasser, Glycerin, Propylenglykol und/oder Sorbitsirup.

9. Zahnpflegekaugummi nach einem der vorhergehenden Ansprüche, **gekennzeichnet durch** mindestens ein Verdickungs-, Stabilisierungs- und/oder Bindemittel, insbesondere Gele, Stärken, Alginate, Öle und/oder Cellulose Gum.

10. Zahnpflegekaugummi nach einem der vorhergehenden Ansprüche, **dadurch gekennzeichnet, dass** der mindestens eine Zuckeralkohol Sorbit, Xylit, Malit und/oder Fructose ist.

11. Zahnpflegekaugummi nach einem der vorhergehenden Ansprüche, **dadurch gekennzeichnet, dass** der mindestens eine aromatische Stoff Menthol und/oder Pfefferminzöl ist.

12. Zahnpflegekaugummi nach einem der vorhergehenden Ansprüche, **gekennzeichnet durch** Süßungsmittel, insbesondere Natriumsaccharin, Aspartam und/oder Acesulfam,

13. Zahnpflegekaugummi nach einem der vorhergehenden Ansprüche, **gekennzeichnet durch** weitere chemische Additive, Farbstoffe und/oder pH-Regulatoren, insbesondere Fluoride, Adstringentien, Desensibilisierungsmittel, Vitamine, Panthenol, Weißpigmente und/oder Natriumhydroxid.

14. Verfahren zur Herstellung eines Zahnpflegekaugummis nach einem der vorhergehenden Ansprüche umfassend die folgenden Schritten:
- Vermischen der pulverförmigen und flüssigen Inhaltsstoffe, insbesondere in einem Konusmischer,
- Kneten des Gemisches bis zur Homogenität,
- Extrusion des Gemisches, und
- Konfektionierung.

15. Verfahren nach Anspruch 14, **dadurch gekennzeichnet, dass** das Gemisch bei einer Endtemperatur von 45°C bis zu Homogenität geknetet wird.

## Claims

1. Dental care chewing gum comprising silver, at least one abrasive, at least one sugar alcohol, gum base and at least one aromatic substance, wherein no zinc in metallic or ionic form is contained and the silver has an average pore size between 1 to 100 µm.

2. Dental care chewing gum comprising silver, at least one abrasive, at least one sugar alcohol, gum base and at least one aromatic substance, wherein no zinc in metallic or ionic form is contained, **characterized in that** the silver is present in the form of an agglomerate with an agglomerate size between 1 to 200 µm, preferably 5 to 100 µm, in particular preferably 5 to 20 µm.

3. The dental care chewing gum according to claim 1, **characterized in that** the silver has an average pore size between 5 to 50 µm, preferably of 10 µm.

4. The dental care chewing gum according to any of the preceding claims, **characterized in that** the silver has no free silver ions.

5. The dental care chewing gum according to any of the preceding claims, **characterized in that** the content of silver is between 0.01 to 1 wt-%, preferably 0.01 to 0.5 wt-%, in particular preferably between 0.01 and 0.1 wt-%.

6. The dental care chewing gum according to any of the preceding claims, **characterized in that** the chewing gum comprises pantothenic acid (vitamin B5) or at least one derivative thereof, in particular dexpanthenol.

7. The dental care chewing gum according to any of the preceding claims, **characterized in that** the at least one abrasive comprises calcium carbonates, calcium phosphates, metaphosphates, silica, aluminum oxides, silicates and/or talcum.

8. The dental care chewing gum according to any of the preceding claims, **characterized by** at least one suspending agent or humectant, in particular water, glycerol, propylene glycol and/or sorbitol syrup.

9. The dental care chewing gum according to any of the preceding claims, **characterized by** at least one thickening, stabilizing and/or binding agent, in particular gels, starches, alginates, oils and/or cellulose gum.

10. The dental care chewing gum according to any of the preceding claims, **characterized in that** the at least one sugar alcohol is sorbitol, xylite, malite and/or fructose.

11. The dental care chewing gum according to any of the preceding claims, **characterized in that** the at least one aromatic substance is menthol and/or peppermint oil.

12. The dental care chewing gum according to any of the preceding claims, **characterized by** sweetening agents, in particular sodium saccharin, aspartame and/or acesulfame.

13. The dental care chewing gum according to any of the preceding claims, **characterized by** further chemical additives, dyes and/or pH regulators, in particular fluorides, astringents, desensitizers, vitamins, panthenol, white pigments and/or sodium hydroxide.

14. A method for producing a dental care chewing gum according to any of the preceding claims, comprising the following steps:
- mixing the powdery and liquid ingredients, in particular in a cone mixer,
- kneading the mixture to homogeneity,
- extruding the mixture, and
- packaging.

15. The method according to claim 14, **characterized in that** the mixture is kneaded to homogeneity at an end temperature of 45°C.

## Revendications

1. Gomme à mâcher pour l'hygiène bucco-dentaire comprenant de l'argent, au moins une matière abrasive, au moins un alcool de sucre, de la gomme base et au moins une substance aromatique, ne contenant pas de zinc sous forme métallique ou ionique et l'argent présentant une taille moyenne de pores comprise entre 1 et 100 µm.

2. Gomme à mâcher pour l'hygiène bucco-dentaire comprenant de l'argent, au moins une matière abrasive, au moins un alcool de sucre, de la gomme base et au moins une substance aromatique, ne contenant pas de zinc sous forme métallique ou ionique, **caractérisée en ce que** l'argent se présente sous forme d'un agglomérat ayant une taille d'agglomérat comprise entre 1 et 200 µm, de préférence entre 5 et 100 µm, d'une manière particulièrement préférée entre 5 et 20 µm.

3. Gomme à mâcher pour l'hygiène bucco-dentaire selon la revendication 1, **caractérisée en ce que** l'argent présente une taille moyenne des pores comprise entre 5 et 50 µm, de préférence de 10 µm.

4. Gomme à mâcher pour l'hygiène bucco-dentaire selon l'une des revendications précédentes, **caractérisée en ce que** l'argent ne comprend pas d'ions argent libres.

5. Gomme à mâcher pour l'hygiène bucco-dentaire selon l'une des revendications précédentes, **caractérisée en ce que** la teneur en argent est comprise entre 0,01 et 1 % en poids, de préférence entre 0,01 et 0,5 % en poids, d'une manière particulièrement préférée entre 0,01 et 0,1 % en poids.

6. Gomme à mâcher pour l'hygiène bucco-dentaire selon l'une des revendications précédentes, **caractérisée en ce que** la gomme à mâcher comprend de l'acide pantothénique (vitamine B5) ou au moins un dérivé de celui-ci, en particulier du dexpanthénol.

7. Gomme à mâcher pour l'hygiène bucco-dentaire selon l'une des revendications précédentes, **caractérisée en ce que** l'au moins un abrasif ou matière abrasive comprend des carbonates de calcium, des phosphates de calcium, des métaphosphates, de la silice, des oxydes d'aluminium, des silicates et/ou du talc.

8. Gomme à mâcher pour l'hygiène bucco-dentaire selon l'une des revendications précédentes, **caractérisée par** au moins une substance de mise en suspension ou au moins un agent humectant, en particulier l'eau, le glycérol, le propylèneglycol et/ou le sirop de sorbitol.

9. Gomme à mâcher pour l'hygiène bucco-dentaire selon l'une des revendications précédentes, **caractérisée par** au moins un agent épaississant, un agent de stabilisation et/ou un liant, en particulier des gels, des amidons, des alginates, des huiles et/ou de la gomme de cellulose.

10. Gomme à mâcher pour l'hygiène bucco-dentaire selon l'une des revendications précédentes, **caractérisée en ce que** l'au moins un alcool de sucre est le sorbitol, le xylitol, le maltitol et/ou le fructose.

11. Gomme à mâcher pour l'hygiène bucco-dentaire selon l'une des revendications précédentes, **caractérisée en ce que** l'au moins une substance aromatique est le menthol et/ou l'essence de menthe poivrée.

12. Gomme à mâcher pour l'hygiène bucco-dentaire selon l'une des revendications précédentes, **caractérisée par** des édulcorants, en particulier la saccharine sodique, l'aspartame et/ou l'acésulfame.

13. Gomme à mâcher pour l'hygiène bucco-dentaire selon l'une des revendications précédentes, **caractérisée par** d'autres additifs chimiques, colorants et/ou régulateurs de pH, en particulier des fluorures, des astringents, des agents de désensibilisation, des vitamines, du panthénol, des pigments blancs et/ou de l'hydroxyde de sodium.

14. Procédé de préparation d'une gomme à mâcher pour l'hygiène bucco-dentaire selon l'une des revendications précédentes, comprenant les étapes suivantes :
- mélange des constituants en poudre et liquides, en particulier dans un mélangeur conique,
- malaxage du mélange jusqu'à homogénéité,
- extrusion du mélange, et
- confectionnement.

15. Procédé selon la revendication 14, **caractérisé en ce que** le mélange est malaxé jusqu'à homogénéité à une température finale de 45°C.
